# NEUE EUROPÄISCHE PATENTSCHRIFT

(11) **EP 0 682 026 B2**
(45) Veröffentlichungstag und Bekanntmachung des Hinweises auf die Entscheidung über den Einspruch: **05.02.2003**
(45) Hinweis auf die Patenterteilung: 12.01.2000
(21) Anmeldenummer: 95106285.0
(22) Anmeldetag: 27.04.1995
(51) Int. Cl.: C07D 475/04, A61K 31/495

(54) **Stabile kristalline (6S)- und (6R)-Tetrahydrofolsäure**
"Stable crystalline (6S)- and (6R)-tetrahydrofolic acid"
"Stable cristalline acide (6S)- et (6R) - tétrahydrofolique"

(30) Priorität: 09.05.1994 CH 144294
(43) Veröffentlichungstag der Anmeldung: 15.11.1995
(73) Patentinhaber: EPROVA Aktiengesellschaft, CH-8200 Schaffhausen (CH)
(72) Erfinder: Müller, Hans Rudolf, CH-8207 Schaffhausen (CH); Moser, Rudolf, CH-8212 Neuhausen (CH); Ulmann, Martin, CH-8447 Dachsen (CH); Ammann, Thomas, CH-8460 Marthalen (CH)

(56) Entgegenhaltungen:
- EP-A- 0 432 441
- EP-A- 0 495 204
- EP-A- 0 600 460
- WO-A-88/08844
- DE-A- 2 323 124

## Beschreibung

Die Erfindung betrifft kristalline N-[4-[[(2-amino-1,4,5,67,8-hexahydro-4-oxo-(6S)- und -(6R)-pteridinyl)methyl]amino]benzoyl]-L-glutaminsäure (im folgenden kristalline (6S)- bzw. (6R)-Tetrahydrofolsäure genannt), deren Verwendung sowie ein Verfahren zu deren Herstellung.

Tetrahydrofolsäure-Derivate enthalten 2 asymmetrische Zentren. Dabei liegt aufgrund der Synthese dieser Derivate aus Folsäure, der N-(Pteroyl)-L-glutaminsäure, das im Glutaminsäure-Rest enthaltene optisch aktive C-Atom in der L-Form vor, während das üblicherweise durch Hydrierung der Doppelbindung in 5,6-Stellung des Pteroyl-Restes entstandene optisch aktive C-Atom in Position 6 in der racemischen, der (6R,S)-Form, vorliegt. Synthetische Derivate der Tetrahydrofolsäure bestehen demnach aus einer 1:1 Mischung von 2 Diastereomeren.

Als Arzneimittel werden Tetrahydrofolate vorwiegend als Calcium-Salz der 5-Formyl-5,6,7,8-tetrahydrofolsäure (Leucovorin) oder der 5-Methyl-5,6,7,8-tetrahydrofolsäure verwendet zur Behandlung von megaloblastischer Folsäure-Anämie, als Antidot zur Verstärkung der Verträglichkeit von Folsäure-Antagonisten, speziell von Aminopterin und Methotrexat in der Krebstherapie ("Antifolate rescue"), zur Verstärkung des therapeutischen Effektes von fluorierten Pyrimidinen und der Behandlung von Autoimmunkrankheiten wie Psoriasis und rheumatischer Arthritis, zur Verstärkung der Verträglichkeit von bestimmten Antiparasitika, etwa Trimethoprim-Sulfamethoxazol sowie zur Verminderung der Toxizität von Dideazatetrahydrofolaten in der Chemotherapie. Ebenfalls dient Tetrahydrofolsäure als Grundsubstanz zur Herstellung diverser Tetrahydrofolsäure-Derivate.

Die direkte Verwendung von Tetrahydrofolsäure als Arzneimittel sowie als Grundsubstanz zur Herstellung diverser Tetrahydrofolsäure-Derivate scheiterte bisher an der Schwierigkeit, Tetrahydrofolsäure mit einer für einen pharmazeutischen Wirkstoff akzeptablen Reinheit herzustellen und an der extremen Instabilität der Tetrahydrofolsäure, wobei insbesondere die hohe Oxidationsempfindlichkeit auffällt [siehe dazu auch A.L. Fitzhugh, Pteridines 4(4), 187-191 (1993)]. Zur Überwindung dieser Instabilität wurden verschiedene Methoden entwickelt, wobei DE-OS-2'323'124 im Zusammenhang mit der vorliegenden Erfindung speziell zu erwähnen ist. Im Bereich der Verfahren zur Herstellung von Tetrahydrofolsäure und im Zusammenhang mit der vorliegenden Erfindung ist EP-600'460 ebenfalls speziell zu erwähnen. Bisher konnte jedoch noch kein technisch gangbares Verfahren gefunden werden, das für die Herstellung von Tetrahydrofolsäure mit einer hohen Reinheit und einer ausreichenden Stabilität geeignet ist und somit die pharmazeutische Verwendung von Tetrahydrofolsäure ermöglichen würde.

Es wurde nun überraschend gefunden, dass sich (6S)- oder (6R)-Tetrahydrofolsäure mit einer hohen chemischen und optischen Reinheit und einer ausgezeichneten Stabilität erhalten lässt, indem optisch reine (6S)- oder optisch reine (6R)-, angereicherte (6S)- oder angereicherte (6R)- Tetrahydrofolsäure kristallisiert wird. Die so erhaltene kristalline (6S)- und/oder (6R)-Tetrahydrofolsäure ermöglicht erstmals deren Verwendung als Arzneimittel oder als Ausgangsmaterial zur technischen Herstellung anderer Tetrahydrofolsäure-Derivate mit einer hohen Reinheit.

Die Kristallisation der (6S)-Tetrahydrofolsäure erfolgt dabei aus einem polaren Medium bei einem pH-Wert von ≥ 3.5, diejenige der (6R)-Tetrahydrofolsäure erfolgt aus einem polaren Medium bei einem pH-Wert von ≥ 2.

Als polares Medium eignen sich vor allem Wasser oder ein Gemisch aus Wasser und einem mit Wasser mischbaren organischen Lösungsmittel wie wasserlöslichen Alkoholen, z.B. Methanol, Ethanol, n-Propanol, iso-Propanol, Ethylenglycol, einer niedrigen aliphatischen wasserlöslichen Carbonsäure, z.B. Ameisensäure, Essigsäure, Milchsäure oder wasserlöslichen Amiden, z.B. Formamid, Dimethylformamid, Dimethylacetamid, 1-Methylpyrrolidon, 2-Methylpyrrolidon, 2-Piperidinon. Es gibt keine besonderen Einschränkungen bezüglich der Art des eingesetzten Lösungsmittels und des Mischungsverhältnisses, da kristalline (6S)-Tetrahydrofolsäure und kristalline (6R)-Tetrahydrofolsäure generell tiefere Löslichkeiten als die entsprechenden amorphen Formen aufweisen.

Zum Starten der Kristallisation der (6S)-Tetrahydrofolsäure eignet sich speziell ein pH-Wert zwischen 3.5 und 6.5. Zum Start der Kristallisation der (6R)-Tetrahydrofolsäure eignet sich speziell ein pH-Wert zwischen 2 und 5.5. Der optimale pH-Wert zum Starten der Kristallisation hängt von den eingesetzten Materialien und dem gewünschten Ziel ab und kann durch einfache Versuche bestimmt werden. Generell gilt, je höher der Salzgehalt in der Ausgangslösung ist, desto tiefer muss der pH-Wert zum Starten der Kristallisation sein, aber auch je tiefer der pH-Wert zum Starten der Kristallisation ist, desto langsamer muss die Kristallisation erfolgen, da sonst bei pH-Werten um 3 amorphe Tetrahydrofolsäure ausfällt. Für die direkte Kristallisation von (6S)-Tetrahydrofolsäure aus einer durch Borhydrid-Reduktion von Folsäure erhaltenen Reaktionslösung wird z. B. zwingend ein pH-Wert zum Starten der Kristallisation von ≤ 4.8 benötigt. Nach dem Starten der Kristallisation kann der pH-Wert verändert werden.
Während der Kristallisation der (6S)-Tetrahydrofolsäure und auch während der Kristallisation der (6R)-Tetrahydrofolsäure erhöht sich der pH-Wert oder er kann durch die Zugabe einer Säure oder eines Puffers konstant gehalten werden. Für die Kristallisation der (6S)-Tetrahydrofolsäure gilt, falls eine optische Anreicherung von (6S)-Tetrahydrofolsäure Ziel der Kristallisation ist, wird ein pH-Wert zwischen 4.5 und 5.5 während der Kristallisation bevorzugt, falls die Herstellung von stabiler kristalliner (6S)-Tetrahydrofolsäure Ziel der Kristallisation ist, wird ein pH-Wert zwischen 3.5 und 4.5 während der Kristallisation bevorzugt. Für die Kristallisation der (6R)-Tetrahydrofolsäure wird unabhängig vom angestrebten Ziel ein pH-Wert zwischen 3.5 und 4.5 während der Kristallisation bevorzugt. Die Kristallisation kann jeweils bei Raumtemperatur, bei erhöhter Temperatur aber auch bei emiedrigter Temperatur durchgeführt werden.
Die Kristallisationsdauer liegt in der Regel zwischen wenigen Minuten und mehreren Tagen. Längere Kristallisationszeiten ergeben in der Regel höhere Reinheiten und stabilere Produkte.

Die Kristallisation der (6S)- und (6R)-Tetrahydrofolsäure erfolgt spontan durch langsames Einstellen des pH-Wertes entweder von einem pH-Wert her, der kleiner ist, als der sich zum Starten der Kristallisation des entsprechenden Isomeren eignende pH-Wert oder vorzugsweise von einem höheren pH-Wert herkommend. Die Kristallisation kann durch Animpfen mit entsprechender kristalliner Tetrahydrofolsäure im sich zum Starten der Kristallisation des entsprechenden Isomeren eignenden pH-Bereiches ausgelöst werden.

Als Ausgangsmaterial der Kristallisation kann sowohl racemische (6R,S)-Tetrahydrofolsäure, angereicherte (6S)- oder (6R)-Tetrahydrofolsäure wie auch amorphe oder kristalline (6S)- oder (6R)-Tetrahydrofolsäure eingesetzt werden. Es eignen sich dabei sowohl isolierte Feststoffe wie z. B. (6R,S)-Tetrahydrofolsäure, (6S)-Tetrahydrofolsäure-Schwefelsäure und Sulfonsäure-Additionssalze hergestellt nach EP-495'204, wie auch in situ durch katalytische Hydrierung oder durch Borhydrid-Reduktion aus Folsäure hergestellte Tetrahydrofolsäure als Ausgangsmaterial. Die Kristallisation der (6R)-Tetrahydrofolsäure kann direkt aus der Mutterlauge der (6S)-Tetrahydrofolsäure-Kristallisation erfolgen. Die Kristallisation beider Isomeren kann sowohl aus einer Lösung, die z. B. durch Einstellen eines pH-Wertes von > 7 oder < 2 erhalten wird, wie auch aus einer Suspension erfolgen.

Durch den Einsatz von amorpher oder teilkristalliner optisch reiner Tetrahydrofolsäure oder deren Salzen als Ausgangsmaterial für die Kristallisation erhält man durch das beschriebene Verfahren kristalline Tetrahydrofolsäure von bisher nie erreichter Reinheit (> 98%) und einer bisher ebenfalls nie erreichten Stabilität.

Die Erfindung betrifft auch die Verwendung kristalliner (6S)- und/oder (6R)-Tetrahydrofolsäure als Bestandteil zur Herstellung von Arzneimitteln oder zur Herstellung anderer Tetrahydrofolsäure-Derivate, da kristalline (6S)- und (6R)-Tetrahydrofolsäure aufgrund ihrer ausgezeichneten Stabilität in fester Form eine zeitlich praktisch unbeschränkt gleichbleibende sehr gute Qualität behält. Ebenso betrifft die Erfindung auch pharmazeutische Zubereitungen enthaltend kristalline (6S)- und/oder (6R)-Tetrahydrofolsäure. Die Herstellung der pharmazeutischen Zubereitung erfolgt nach bekannten Verfahren wie z. B. Lyophilisierung. Die Anwendung erfolgt analog zur Anwendung von bekannten Substanzen aus dem Gebiet der Tetrahydrofolate wie z. B. 5-Formyl-5,6,7,8-tetrahydrofolsäure.

Im weiteren betrifft die Erfindung ein Verfahren zur Trennung von (6R,S)-Tetrahydrofolsäure in die beiden Diastereomeren (6S)- und (6R)-Tetrahydrofolsäure durch fraktionierte Kristallisation. Dieses Verfahren ist sehr einfach und ergiebig. Man erhält bereits bei der ersten Kristallisation einer rohen racemischen (6R,S)-Tetrahydrofolsäure kristalline (6S)-Tetrahydrofolsäure mit einem (6S)-Anteil von über 75% in Ausbeuten von über 70% und kristalline (6R)-Tetrahydrofolsäure mit einem (6R)-Anteil von über 80% in Ausbeuten von über 50%. Durch weitere Kristallisationen unter analogen Bedingungen kann kristalline (6S)- und (6R)-Tetrahydrofolsäure mit einer Isomerenreinheit von über 95% erhalten werden.

(6R)- oder (6S)-Tetrahydrofolsäure kann auch direkt ohne Isolierung zur Herstellung anderer Tetrahydrofolsäure-Derivate verwendet werden. So kann z.B. durch die Zugabe von Formaldehyd zu einer Lösung von (6R)-Tetrahydrofolsäure sehr einfach angereicherte 5,10-Methylen-(6S)-tetrahydrofolsäure hergestellt werden.

### Beispiele zur Illustrierung der Erfindung

Die in den Beispielen angegebenen Gehalte an Tetrahydrofolsäure und die Isomerenanteile wurden jeweils mit HPLC bestimmt. Alle Tetrahydrofolsäure-Gehalte sind auf wasserfreie Basis bezogen.

### Beispiel 1 (Stabilitäten)

Zur Stabilitätsbestimmung der kristallinen (6S)- und (6R)-Tetrahydrofolsäure wurden die Substanzen zusammen mit Vergleichsmustern unter Stressbedingungen bei 60°C unter Luft gelagert. In periodischen Abständen wurde der verbleibende Gehalt an Tetrahydrofolsäure gemessen und im Vergleich zum Ausgangswert angegeben.

| | **Belastungszeit in Tagen bei 60°C unter Luft** | | | | | | | |
|---|---|---|---|---|---|---|---|---|
| | **0** | **2** | **6** | **13** | **21** | **28** | **57** | **360** |
| kristalline (6S)-Tetrahydrofolsäure | 100.0% | 100.1% | 102.5% | 98.7% | 103.6% | 103.1% | 101.2% | 93.3% |
| kristalline (6R)-Tetrahydrofolsäure | 100.0% | 98.4% | 96.1% | 93.3% | 92.7% | | 82.0% | |
| "kristalline (6R,S)-Tetrahydrofolsäure nach Yamanouchi" | 100.0% | 83.6% | | 48.6% | 31.0% | | 13.4% | |
| amorphe (6S)-Tetra hydrofolsäure | 100.0% | 60.4% | | 13.7% | 7.9% | | | |
| amorphe (6R)-Tetra hydrofolsäure | 100.0% | 70.5% | | 29.1% | 21.6% | | 9.8% | |
| amorphe (6R,S) Tetrahydrofolsäure | 100.0% | 53.4% | | 17.4% | 13.2% | | | |

Kristalline (6S)- und (6R)-Tetrahydrofolsäure sind auch nach längerer Belastungszeit bei 60°C unter Luft noch sehr hell, fast weiss. Im Gegensatz dazu verfärben sich die anderen zum Vergleich angeführten Produkte rasch sehr stark.

Die für die Stabilitätsversuche eingesetzten Substanzen wurden wie folgt hergestellt:
- kristalline (6S)-Tetrahydrofolsäure
   nach Beispiel 6 der vorliegenden Patentanmeldung
- kristalline (6R)-Tetrahydrofolsäure
   nach Beispiel 9 der vorliegenden Patentanmeldung
- "kristalline (6R,S)-Tetrahydrofolsäure nach Yamanouchi"
   nach DE-OS 2'323'124, Beispiel 3
- amorphe (6S)-Tetrahydrofolsäure
   (6S)-Tetrahydrofolsäure wird in Essigsäure gelöst und mit Diethylether gefällt
- amorphe (6R)-Tetrahydrofolsäure
   (6R)-Tetrahydrofolsäure wird in Essigsäure gelöst und mit Diethylether gefällt.
- amorphe (6R,S)-Tetrahydrofolsäure
   (6R,S)-Tetrahydrofolsäure wird in Essigsäure gelöst und mit Diethylether gefällt.

### Beispiel 2 (Pulverröntgendiagramme)

Zur Charakterisierung der strukturellen Eigenschaften (Kristallinität) der kristallinen (6S)- und (6R)-Tetrahydrofolsäure wurden von diesen Substanzen zusammen mit Vergleichsmustern unter gleichen Bedingungen Pulverröntgendiagramme (Beugungsspektren) aufgenommen.

Kristalline (6S)- und kristalline (6R)-Tetrahydrofolsäure ergeben beide gut aufgelöste, unterschiedliche Spektren mit scharfen Banden und niedrigem Untergrund. Die Spektren weisen auf hohe kristalline Anteile hin. Im Gegensatz dazu ergibt "kristalline (6R,S)-Tetrahydrofolsäure nach Yamanouchi" ein schlecht aufgelöstes Spektrum mit unscharfen Banden (diffuse Maxima) und hohem Untergrund. Dieses Spektrum weist auf überwiegend vorliegende amorphe (6R,S)-Tetrahydrofolsäure mit nur einem geringen kristallinen Anteil hin.

Für die Messung der Pulverröntgendiagramme eingesetzte Substanzen wurden wie folgt hergestellt:
- kristalline (6S)-Tetrahydrofolsäure
   nach Beispiel 6 der vorliegenden Patentanmeldung
- kristalline (6R)-Tetrahydrofolsäure
   nach Beispiel 9 der vorliegenden Patentanmeldung
- "kristalline (6R,S)-Tetrahydrofolsäure nach Yamanouchi"
   nach DE-OS 2'323'124, Beispiel 3

### Beispiel 3

a) 4 g (6R,S)-Tetrahydrofolsäure werden in 16 ml Wasser aufgeschlämmt und mit Ammoniak 25% auf pH 9 gestellt.
Die entstandene Lösung wird bei 50°C mit Salzsäure auf pH 5 und danach mit Natronlauge langsam auf den gewünschten pH-Wert eingestellt. Beim angegebenen pH-Wert werden jeweils 2 ml-Proben entnommen, abgenutscht und mit wenig Wasser gewaschen.

| **pH** | **Menge** | **(6S)-Anteil** |
|---|---|---|
| pH 5.5 | 0.03 g | 87.8% |
| pH 6.0 | 0.06 g | 87.8% |
| pH 6.4 | 0.02 g | 88.6% |

b) 4 g (6R,S)-Tetrahydrofolsäure werden in 16 ml Wasser aufgeschlämmt und mit Ammoniak 25% auf pH 9 gestellt.
Die entstandene Lösung wird bei 50°C mit Salzsäure auf pH 5 und danach mit Salzsäure langsam auf den gewünschten pH-Wert eingestellt. Beim angegebenen pH-Wert werden jeweils 2 ml-Proben entnommen, abgenutscht und mit wenig Wasser gewaschen.

| **pH** | **Menge** | **(6S)-Anteil** |
|---|---|---|
| pH 4.8 | 0.09 g | 72.7% |
| pH 4.5 | 0.15 g | 57.9% |
| pH 4.2 | 0.27 g | 51.8% |

c) 4 g (6R,S)-Tetrahydrofolsäure werden in 16 ml Wasser aufgeschlämmt und mit Ammoniak 25% auf pH 9 gestellt.
Die entstandene Lösung wird bei 50°C mit Salzsäure auf pH 5 und danach mit Salzsäure langsam auf den gewünschten pH-Wert eingestellt. Beim angegebenen pH-Wert werden jeweils 2 ml-Proben entnommen, abgenutscht und mit wenig Wasser gewaschen.

| **pH** | **Menge** | **(6S)-Antell** |
|---|---|---|
| pH 4.1 | 0.16 g | 56.2% |
| pH 3.8 | 0.10 g | 52.2% |
| pH 3.5 | 0.22 g | 51.8% |
| pH 3.0 | 0.12g | 51.6% |

d) 10 g (6R,S)-Tetrahydrofolsäure werden in 80 ml Wasser aufgeschlämmt und mit Salzsäure 1N auf pH 1.3 gestellt. Die entstandene Lösung wird bei Raumtemperatur mit Ammoniak 1.8 N langsam auf den gewünschten pH-Wert eingestellt. Beim angegebenen pH-Wert werden jeweils 2 ml-Proben entnommen, abgenutscht und mit wenig Wasser gewaschen.

| **pH** | **Menge** | **(6S)-Anteil** |
|---|---|---|
| pH 2.0 | 0.03 g | 50.3% |
| pH 2.3 | 0.13 g | 50.5% |
| pH 2.5 | 0.12 g | 49.3% |
| pH 2.8 | 0.22 g | 50.8% |
| pH 3.1 | 0.17 g | 49.5% |
| pH 3.5 | 0.21 g | 51.5% |
| pH 4.0 | 0.14 g | 59.1% |
| pH 4.5 | 0.16 g | 56.1% |
| pH 5.1 | 0.22 g | 72.7% |
| pH 5.5 | 0.20 g | 70.9% |

Die in den Tabellen a) bis d) aufgeführten Werte entsprechen nicht den optimalen Verfahrensparametern, da alle Versuche zum Zweck der besseren Vergleichbarkeit gleich durchgeführt wurden.

### Beispiel 4

Je 5 g (6R,S)-Tetrahydrofolsäure werden in 50 ml Wasser aufgeschlämmt und 5 Tage bei Raumtemperatur bzw. bei 40°C stehen gelassen. Nach dem Abnutschen (Filtrationstemperatur = Kristallisationstemperatur) und nachwaschen erhält man:

| | **RT** | | **40°C** | |
|---|---|---|---|---|
| | **Menge** | **(6S)-Anteil** | **Menge** | **(6S)-Anteil** |
| pH 3.1 ¹⁾ | 4.2 g | 52.5% | 4.5 g | 52.2% |
| pH 4.2 ²⁾ | 3.5 g | 58.9% | 3.9 g | 59.3% |
| pH 5.1 ²⁾ | 1.8 g | 82.1% | 1.5 g | 81.0% |

| | | | | |
|---|---|---|---|---|
| ¹⁾ pH-Wert beim Aufschlämmen der (6R,S)-Tetrahydrofolsaure ohne Korrektur, analog DE-OS 2'323'124, Beispiel 3 | | | | |
| ²⁾ mit Natronlauge auf gewünschten pH-Wert eingestellt | | | | |

Die in der Tabelle aufgeführten Werte entsprechen nicht den optimalen Verfahrensparametern, da alle Versuche zum Zweck der besseren Vergleichbarkeit gleich durchgeführt wurden.

### Beispiel 5

40 g (6R,S)-Tetrahydrofolsäure werden in 160 ml Wasser aufgeschlämmt und mit Ammoniak 25% auf pH 9.3 gestellt. Die entstandene Lösung wird bei 50°C mit Salzsäure langsam auf pH 5.1 gestellt und während der folgenden Kristallisation zwischen pH 5.1 und 5.2 gehalten. Nach erfolgter Kristallisation wird auf 0-5°C abgekühlt, druckfiltriert und mit Wasser gewaschen.

Man erhält 19 g kristalline (6S)-Tetrahydrofolsäure mit einem chemischen Gehalt von 95.9% und einem (6S)-Anteil von 80.5%.

Aus der einen Hälfte der Mutterlauge erhält man durch Fällen mit Ethanol 1.1 g angereicherte, amorphe (6R)-Tetrahydrofolsäure mit einem chemischen Gehalt von 63.3% und einem (6R)-Anteil von 75.9% und aus der anderen Hälfte der Mutterlauge erhält man durch rasches Einstellen des pH-Wertes auf 3.5 mit Salzsäure 6.3 g angereicherte, amorphe (6R)-Tetrahydrofolsäure mit einem chemischen Gehalt von 84.8% und einem (6R)-Anteil von 75.9%.

### Beispiel 6

60 g (6S)-Tetrahydrofolsäure-Benzolsulfonsäure-Additionssalz mit einem (6S)-Anteil von 99.9% hergestellt nach EP-495'204 werden in 240 ml Wasser aufgeschlämmt und mit 63 ml Ammoniak 1.8N oder 55.2 ml Natronlauge 2N auf pH 5.5 eingestellt. Der pH-Wert wird bei 5.5 gehalten. Die weisse, dicke Suspension wird anschliessend mit Natronlauge 30% auf pH 9.3 eingestellt und die entstandene klare Lösung wird auf 50°C erwärmt.

Nach anschliessendem langsamen Einstellen des pH-Wertes mit Salzsäure auf pH 5.2 erhält man nach Animpfen mit kristalliner (6S)-Tetrahydrofolsäure 43.0 g kristalline (6S)-Tetrahydrofolsäure mit einem chemischen Gehalt von 96.8% und einem (6S)-Anteil von 99.9%.

Durch Lösen von 40 g der erhaltenen kristallinen (6S)-Tetrahydrofolsaure in 160 ml Wasser bei pH 9 und anschliessendem langsamen Einstellen des pH-Wertes mit Salzsäure auf 4.2 erhält man nach Animpfen mit kristalliner (6S)-Tetrahydrofolsäure 32.5 g kristalline (6S)-Tetrahydrofolsäure mit einem chemischen Gehalt von 98.5% und einem (6S)-Anteil von 100.0%.

Durch weitere Umkristallisationen bei pH 4.2 erhält man kristalline (6S)-Tetrahydrofolsäure mit einem chemischen Gehalt von > 99% und einem (6S)-Anteil von 100.0%.

Die Löslichkeit der so erhaltenen kristallinen (6S)-Tetrahydrofolsäure bei Raumtemperatur in Wasser beträgt 0.0022%.

### Beispiel 7

40 g (6R,S)-Tetrahydrofolsäure werden in 160 ml Wasser und 40 ml Methanol aufgeschlämmt und mit Ammoniak 25% auf pH 9.1 gestellt. Die entstandene Lösung wird mit Salzsäure bei 50°C langsam auf pH 5.1 gestellt und während der folgenden Kristallisation zwischen pH 5.1 und 5.2 gehalten. Nach erfolgter Kristallisation wird eine Teilprobe von 20 ml bei 50°C abgenutscht und mit Wasser/Methanol gewaschen. Man erhält 1.3 g kristalline (6S)-Tetrahydrofolsäure mit einem chemischen Gehalt von 96.1% und einem (6S)-Anteil von 83.0%.
Die Hauptmenge wird auf 0-5°C abgekühlt, druckfiltriert und mit Wasser/Methanol gewaschen. Man erhält weitere 18.6 g kristalline (6S)-Tetrahydrofolsäure mit einem chemischen Gehalt von 90.9% und einem (6S)-Anteil von 67.1%.

### Beispiel 8

60 g Folsäure werden in 240 ml Wasser suspendiert und mit Natronlauge 30% auf pH 11.5 eingestellt. Die entstandene Lösung wird mit 30 g Natriumborhydrid in 120 ml Wasser und 12 g Natronlauge 30% bei 70°C reduziert. Nach ca 5 Stunden Reaktionszeit wird die Reaktionsmischung mit 180 ml Wasser verdünnt und mit Salzsäure langsam auf pH 4.5 eingestellt. Während der folgenden Kristallisation steigt der pH auf ca 5.5. Die Suspension wird bei 0-5°C druckfiltriert und mit wenig Wasser gewaschen.

Man erhält 25.5 g kristalline (6S)-Tetrahydrofolsäure mit einem chemischen Gehalt von 94.4% und einem (6S)-Anteil von 82.7%. Der Wassergehalt nach dem Trocknen beträgt 4.0%.

Durch Lösen von 20 g der erhaltenen kristallinen (6S)-Tetrahydrofolsäure in 80 ml Wasser bei pH 9 und anschliessendem langsamen Einstellen des pH-Wertes mit Salzsäure auf 5.1 erhält man nach Animpfen mit kristalliner (6S)-Tetrahydrofolsäure 4.5 g kristalline (6S)-Tetrahydrofolsäure mit einem chemischen Gehalt von 94.0% und einem (6S)-Anteil von 94.7%. Der Wassergehalt nach dem Trocknen beträgt 1.8%.

### Beispiel 9

50 g amorphe (6R)-Tetrahydrofolsäure mit einem (6R)-Anteil von 99.4% werden in 600 ml Wasser aufgeschlämmt und mit Ammoniak 25% auf pH 9.0 eingestellt. Die entstandene klare Lösung wird auf 50°C erwärmt.

Nach anschliessendem langsamen Einstellen des pH-Wertes mit Salzsäure auf 4.4 und halten dieses Wertes erhält man 42.0 g kristalline (6R)-Tetrahydrofolsäure mit einem chemischen Gehalt von 96.2% und einem (6R)-Anteil von 99.5%.
Die Löslichkeit der so erhaltenen kristallinen (6R)-Tetrahydrofolsäure bei Raumtemperatur in Wasser beträgt 0.014%.

Durch weitere Umkristallisationen bei pH 4.4 erhält man kristalline (6R)-Tetrahydrofolsäure mit einem chemischen Gehalt von > 98% und einem (6R)-Anteil von > 99.5%.

### Beispiel 10

40 g (6R,S)-Tetrahydrofolsäure werden in 160 ml Wasser aufgeschlämmt und mit Ammoniak 25% auf pH 9.3 gestellt. Die entstandene Lösung wird bei 50°C mit Salzsäure langsam auf pH 5.1 gestellt und während der folgenden Kristallisation zwischen pH 5.1 und 5.2 gehalten. Nach erfolgter Kristallisation wird auf Raumtemperatur abgekühlt, druckfiltriert und mit Wasser gewaschen.

Man erhält 18.2 g kristalline (6S)-Tetrahydrofolsäure mit einem chemischen Gehalt von 94.0% und einem (6S)-Anteil von 77.8%.

Die Mutterlauge der (6S)-Tetrahydrofolsäure-Kristallisation wird wieder auf 50°C erwärmt und mit Salzsäure langsam auf pH 4.4 gestellt und während der folgenden. Kristallisation der (6R)-Tetrahydrofolsäure zwischen pH 4.0 und 4.5 gehalten. Nach erfolgter Kristallisation wird auf Raumtemperatur abgekühlt, druckfiltriert und mit Wasser gewaschen.

Man erhält 12 g kristalline (6R)-Tetrahydrofolsäure mit einem chemischen Gehalt von 78.0% und einem (6R)-Anteil von 74.8%.

### Beispiel 11

Analog den in EP-600'460 Beispiel 2 beschriebenen Bedingungen wurde ausgehend von racemischer Tetrahydrofolsäure das beschriebene Verfahren bei pH 5.2 und 45°C wiederholt durchgeführt. Die dabei erhaltenen Produkte wurden auf chemische und optische Reinheit untersucht. Ebenso wurde die chemische Gesamtausbeute bis zur jeweiligen Stufe protokolliert.

| | **6S-Anteil** | **Gehalt g/g** | **Reinheit 6S-Anteil • Gehalt** | **Gesamtausbeute** |
|---|---|---|---|---|
| Ausgangsmaterial | 50 % | 89.6% | 44.8% | 100 % |
| 1. Durchlauf | 80.5% | 96.9% | 78.0% | 53.5% |
| 2. Durchlauf | 90.0% | 97.3% | 87.6% | 45.4% |
| 3. Durchlauf | 94.4% | 97.4% | 91.9% | 42.0% |
| 4. Durchlauf | 96.4% | 96.7% | 93.2% | 38.9% |
| 5. Durchlauf | 97.7% | 96.2% | 94.0% | 35.2% |
| 6. Durchlauf | 98.4% | 95.6% | 94.1% | 32.2% |
| 7. Durchlauf | 98.9% | 96.0% | 94.9% | 28.6% |

Wie klar aus diesen Angaben ersichtlich ist, kann durch mehrfaches Anwenden dieses Verfahrens wohl der (6S)-Anteil im Verfahrensprodukt gesteigert werden, allerdings muss auch eine durch die mehrfache Durchführung des Verfahrens bedingte Abnahme des Produktgehaltes in Kauf genommen werden. Die Herstellung eines Produkts mit einer Reinheit von über 98% ist daher auch durch mehrfaches Anwenden dieses Verfahrens nicht möglich.

## Patentansprüche (Patentansprüche für folgende(n) Vertragsstaat(en): AT, BE, DK, GR, IE, LU, MC, NL, PT, SE)

1. Kristalline (6S)-Tetrahydrofolsäure.

2. Kristalline (6R)-Tetrahydrofolsäure.

3. Verfahren zur Herstellung von kristalliner (6S)- oder (6R)-Tetrahydrofolsäure, **dadurch gekennzeichnet, dass** man (6S)- oder (6R)-Tetrahydrofolsäure kristallisiert.

4. Verfahren zur Herstellung von kristalliner (6S)-Tetrahydrofolsäure, **dadurch gekennzeichnet, dass** man (6S)- oder (6R,S)-Tetrahydrofolsäure in einem polaren Medium bei einem pH-Wert von ≥ 3.5 kristallisiert.

5. Verfahren zur Herstellung von kristalliner (6R)-Tetrahydrofolsäure, **dadurch gekennzeichnet, dass** man (6R)- oder (6R,S)-Tetrahydrofolsäure in einem polaren Medium bei einem pH-Wert von ≥ 2 kristallisiert.

6. Verfahren nach Anspruch 4 oder 5, wobei als polares Medium Wasser oder ein Gemisch aus Wasser und einem polaren wasserlöslichen organischen Lösungsmittel verwendet wird und die Kristallisation aus einer Lösung oder aus einer Suspension erfolgt.

7. Verfahren nach Anspruch 4 oder 6, wobei zum Starten der Kristallisation der (6S)-Tetrahydrofolsäure ein pH-Wert zwischen 3.5 und 6.5 eingestellt wird.

8. Verfahren nach Anspruch 5 oder 6, wobei zum Starten der Kristallisation der (6R)-Tetrahydrofolsäure ein pH-Wert zwischen 2 und 5.5 eingestellt wird.

9. Verfahren nach Anspruch 3, 4, 5, 6, 7 oder 8, **dadurch gekennzeichnet, dass** der pH-Wert während der Kristallisation konstant gehalten wird.

10. Verfahren nach Anspruch 3, 4, 5, 6, 7, 8 oder 9, wobei (6S)-Tetrahydrofolsäure durch fraktionierte Kristallisation abgetrennt und aus der zurückbleibenden Mutterlauge (6R)-Tetrahydrofolsäure isoliert wird.

11. Verwendung von kristalliner (6S)- oder (6R)-Tetrahydrofolsäure als Bestandteil zur Herstellung von Arzneimitteln.

12. Verwendung von kristalliner (6S)- oder (6R)-Tetrahydrofolsäure hergestellt nach Anspruch 3, 4, 5, 6, 7, 8, 9 oder 10 als Bestandteil zur Herstellung von Arzneimitteln.

13. Pharmazeutische Zubereitung enthaltend kristalline (6S)- oder (6R)-Tetrahydrofolsäure.

## Patentansprüche (Patentansprüche für folgende(n) Vertragsstaat(en): CH, DE, ES, FR, GB, IT, LI)

1. Kristalline (6R)-Tetrahydrofolsäure.

2. Verfahren zur Herstellung von kristalliner (6R)-Tetrahydrofolsäure, **dadurch gekennzeichnet, dass** man (6R)- oder (6R,S)-Tetrahydrofolsäure kristallisiert.

3. Verfahren zur Herstellung von kristalliner (6S)-Tetrahydrofolsäure, **dadurch gekennzeichnet, dass** man (6S)- oder (6R,S)-Tetrahydrofolsäure in einem polaren Medium bei einem pH-Wert zwischen 3.5 und 4.5 kristallisiert.

4. Verfahren zur Herstellung von kristalliner (6R)-Tetrahydrofolsäure, **dadurch gekennzeichnet, dass** man (6R)- oder (6R,S)-Tetrahydrofolsäure in einem polaren Medium bei einem pH-Wert von ≥ 2 kristallisiert.

5. Verfahren nach Anspruch 3 oder 4, wobei als polares Medium Wasser oder ein Gemisch aus Wasser und einem polaren wasserlöslichen organischen Lösungsmittel verwendet wird und die Kristallisation aus einer Lösung oder aus einer Suspension erfolgt.

6. Verfahren nach Anspruch 3 oder 5, wobei zum Starten der Kristallisation der (6S)-Tetrahydrofolsäure ein pH-Wert zwischen 3.5 und 6.5 eingestellt wird.

7. Verfahren nach Anspruch 4 oder 5, wobei zum Starten der Kristallisation der (6R)-Tetrahydrofolsäure ein pH-Wert zwischen 2 und 5.5 eingestellt wird.

8. Verfahren nach Anspruch 2, 3, 4, 5, 6 oder 7, **dadurch gekennzeichnet, dass** der pH-Wert während der Kristallisation konstant gehalten wird.

9. Verfahren nach Anspruch 2, 3, 4, 5, 6, 7 oder 8, wobei (6S)-Tetrahydrofolsäure durch fraktionierte Kristallisation abgetrennt und aus der zurückbleibenden Mutterlauge (6R)-Tetrahydrofolsäure isoliert wird.

10. Verwendung von kristalliner (6S)- oder (6R)-Tetrahydrofolsäure als Bestandteil zur Herstellung von Arzneimitteln.

11. Verwendung von kristalliner (6S)- oder (6R)-Tetrahydrofolsäure hergestellt nach Anspruch 2, 4, 5, 6, 7, 8 oder 9 als Bestandteil zur Herstellung von Arzneimitteln.

12. Pharmazeutische Zubereitung enthaltend kristalline (6S)- oder (6R)-Tetrahydrofolsäure.

## Claims (Claims for the following Contracting State(s): AT, BE, DK, GR, IE, LU, MC, NL, PT, SE)

1. Crystalline (6S)-tetrahydrofolic acid.

2. Crystalline (6R)-tetrahydrofolic acid.

3. Process for the preparation of crystalline (6S)-and/or (6R)-tetrahydrofolic acid, **characterized in that** (6S)- or (6R)- tetrahydrofolic acid is crystallized.

4. Process for the preparation of crystalline (6S)-tetrahydrofolic acid, **characterized in that** (6S)- or (6R,S)-tetrahydrofolic acid is crystallized in a polar medium at a pH of ≥ 3.5.

5. Process for the preparation of crystalline (6R)-tetrahydrofolic acid, **characterized in that** (6R)- or (6R,S)-tetrahydrofolic acid is crystallized in a polar medium at a pH of ≥ 2.

6. Process according to Claim 4 or 5, in which water or a mixture of water and a polar water-soluble organic solvent is used as the polar medium and crystallization is effected from a solution or a suspension.

7. Process according to Claim 4 or 6, wherein the pH is brought to between 3.5 and 6.5 to initiate crystallization of (6S)-tetrahydrofolic acid.

8. Process according to Claim 5 or 6, wherein the pH is brought to between 2 and 5.5 to initiate crystallization of (6R)-tetrahydrofolic acid.

9. Process according to Claim 3, 4, 5, 6, 7 or 8, **characterized in that** the pH is maintained constant during crystallization.

10. Process according to Claim 3, 4, 5, 6, 7, 8 or 9, wherein (6S)-tetrahydrofolic acid is separated off by fractional crystallization and (6R)-tetrahydrofolic acid is isolated from the remaining mother liquor.

11. Use of crystalline (6S)- or (6R)-tetrahydrofolic acid as a component for the preparation of pharmaceuticals.

12. Use of crystalline (6S)- or (6R)-tetrahydrofolic acid, prepared according to Claim 3, 4, 5, 6, 7, 8, 9 or 10 as a component for the preparation of pharmaceuticals.

13. Pharmaceutical preparation comprising crystalline (6S)- or (6R)-tetrahydrofolic acid.

## Claims (Claims for the following Contracting State(s): CH, DE, ES, FR, GB, IT, LI)

1. Crystalline (6R)-tetrahydrofolic acid.

2. Process for the preparation of crystalline (6R)-tetrahydrofolic acid, **characterized in that** (6R)- or (6R,S)-tetrahydrofolic acid is crystallized.

3. Process for the preparation of crystalline (6S)-tetrahydrofolic acid, **characterized in that** (6S)- or (6R,S)(6R,S)-tetrahydrofolic acid is crystallized in a polar medium at a pH of between 3.5 and 4.5.

4. Process for the preparation of crystalline (6R)-tetrahydrofolic acid, **characterized in that** (6R)- or (6R,S)-tetrahydrofolic acid is crystallized in a polar medium at a pH of ≥ 2.

5. Process according to Claim 3 or 4, in which water or a mixture of water and a polar water-soluble organic solvent is used as the polar medium and crystallization is effected from a solution or a suspension.

6. Process according to Claim 3 or 5, wherein the pH is brought to between 3.5 and 6.5 to initiate crystallization of (6S)-tetrahydrofolic acid.

7. Process according to Claim 4 or 5, wherein the pH is brought to between 2 and 5.5 to initiate crystallization of (6R)-tetrahydrofolic acid.

8. Process according to Claim 2, 3, 4, 5, 6 or 7, **characterized in that** the pH is maintained constant during crystallization.

9. Process according to Claim 2, 3, 4, 5, 6, 7 or 8, wherein (6S)-tetrahydrofolic acid is separated off by fractional crystallization and (6R)-tetrahydrofolic acid is isolated from the remaining mother liquor.

10. Use of crystalline (6S)- or (6R)-tetrahydrofolic acid as a component for the preparation of pharmaceuticals.

11. Use of crystalline (6S)- or (6R)-tetrahydrofolic acid, prepared according to Claim 2, 4, 5, 6, 7, 8 or 9 as a component for the preparation of pharmaceuticals.

12. Pharmaceutical preparation comprising crystalline (6S)- or (6R)-tetrahydrofolic acid.

## Revendications (Revendications pour l'(les) Etat(s) contractant(s) suivant(s): AT, BE, DK, GR, IE, LU, MC, NL, PT, SE)

1. Acide (6S)-tétrahydrofolique cristallin.

2. Acide (6R)-tétrahydrofolique cristallin.

3. Procédé pour la préparation d'acide (6S)- ou (6R)-tétrahydrofolique cristallin, **caractérisé en ce que** l'on fait cristalliser l'acide (6S)- ou (6R)-tétrahydrofolique.

4. Procédé pour la préparation d'acide (6S)-tétrahydrofolique **caractérisé en ce que** l'on fait cristalliser l'acide (6S)- ou (6R,S)-tétrahydrofolique dans un milieu polaire à une valeur de pH de ≥ 3,5.

5. Procédé pour la préparation d'acide (6R)-tétrahydrofolique, **caractérisé en ce que** l'on fait cristalliser l'acide (6R)- ou (6R,S)-tétrahydrofolique dans un milieu polaire à une valeur de pH de ≥ 2.

6. Procédé selon la revendication 4 ou 5, dans lequel on utilise comme milieu polaire l'eau ou un mélange d'eau et d'un solvant organique hydrosoluble polaire et on réalise la cristallisation dans une solution ou dans une suspension.

7. Procédé selon la revendication 4 ou 6, dans lequel on ajuste pour le démarrage de la cristallisation de l'acide (6S)-tétrahydrofolique une valeur de pH entre 3,5 et 6,5.

8. Procédé selon la revendication 5 ou 6, dans lequel on ajuste pour le démarrage de la cristallisation de l'acide (6R)-tétrahydrofolique une valeur de pH entre 2 et 5,5.

9. Procédé selon la revendication 3, 4, 5, 6, 7 ou 8, **caractérisé en ce que** la valeur du pH est maintenue constante pendant la cristallisation.

10. Procédé selon la revendication 3, 4, 5, 6, 7, 8 ou 9, dans lequel on sépare l'acide (6S)-tétrahydrofolique par cristallisation fractionnée et on isole l'acide (6R)-tétrahydrofolique de la solution mère restante.

11. Utilisation d'acide (6S)- ou (6R)-tétrahydrofolique cristallin comme constituant pour la préparation de médicaments.

12. Utilisation d'acide (6S)- ou (6R)-tétrahydrofolique cristallin préparé selon la revendication 3, 4, 5, 6, 7, 8, 9 ou 10 comme constituant pour la préparation de médicaments.

13. Préparation pharmaceutique contenant l'acide (6S)- ou (6R)-tétrahydrofolique cristallin.

## Revendications (Revendications pour l'(les) Etat(s) contractant(s) suivant(s): CH, DE, ES, FR, GB, IT, LI)

1. Acide (6R)-tétrahydrofolique cristallin.

2. Procédé pour la préparation d'acide (6R)-tétrahydrofolique cristallin, **caractérisé en ce que** l'on fait cristalliser l'acide (6R)- ou (6R,S)-tétrahydrofolique.

3. Procédé pour la préparation d'acide (6S)-tétrahydrofolique cristallin, **caractérisé en ce que** l'on fait cristalliser l'acide (6S)- ou (6R,S)-tétrahydrofolique dans un milieu polaire à une valeur de pH entre 3,5 et 4,5.

4. Procédé pour la préparation d'acide (6R)-tétrahydrofolique, **caractérisé en ce que**, l'on fait cristalliser l'acide (6R)- ou (6R,S)-tétrahydrofolique dans un milieu polaire à une valeur de pH de ≥ 2.

5. Procédé selon la revendication 3 ou 4, dans lequel on utilise comme milieu polaire l'eau ou un mélange d'eau et d'un solvant organique hydrosoluble polaire et on réalise la cristallisation dans une solution ou dans une suspension.

6. Procédé selon la revendication 3 ou 5, dans lequel on ajuste pour le démarrage de la cristallisation de l'acide (6S)-tétrahydrofolique une valeur de pH entre 3,5 et 6,5.

7. Procédé selon la revendication 4 ou 5, dans lequel on ajuste pour le démarrage de la cristallisation de l'acide (6R)-tétrahydrofolique une valeur de pH entre 2 et 5,5.

8. Procédé selon la revendication 2, 3, 4, 5, 6 ou 7, **caractérisé en ce que** la valeur du pH est maintenue constante pendant la cristallisation.

9. Procédé selon la revendication 2, 3, 4, 5, 6, 7 ou 8, dans lequel on sépare l'acide (6S)-tétrahydrofolique par cristallisation fractionnée et on isole l'acide (6R)-tétrahydrofolique de la solution mère restante.

10. Utilisation d'acide (6S)- ou (6R)-tétrahydrofolique cristallin comme constituant pour la préparation de médicaments.

11. Utilisation d'acide (6S)- ou (6R)-tétrahydrofolique cristallin préparé selon la revendication 2, 4, 5, 6, 7, 8 ou 9 comme constituant pour la préparation de médicaments.

12. Préparation pharmaceutique contenant l'acide (6S)-ou (6R)-tétrahydrofolique cristallin.
